# EUROPÄISCHE PATENTANMELDUNG

(11) **EP 4 321 109 A1**
(43) Veröffentlichungstag der Anmeldung: **14.02.2024**
(21) Anmeldenummer: 23190385.7
(22) Anmeldetag: 08.08.2023
(51) Int. Cl.: A61B 17/16

(54) **MEDIZINISCHES MOTORHANDSTÜCK MIT WINKELSTELLRING UND MEDIZINISCHES HANDINSTRUMENT**

(30) Priorität: 09.08.2022 DE 102022119983
(71) Anmelder: Aesculap AG, 78532 Tuttlingen (DE)
(72) Erfinder: BUERK, Andre, 78056 Villingen-Schwenningen (DE); VOGLER, Aaron, 88637 Leibertingen (DE)
(74) Vertreter: Winter, Brandl - Partnerschaft mbB

(57) **Zusammenfassung**

Die Offenbarung betrifft ein medizinisches Motorhandstück (2) zum Antreiben eines distalen Endeffektors (8, 22), mit einem Handgriffabschnitt (4), einem vorzugsweise hülsenförmigen Bedienelement (12), das um eine Handgrifflängsachse drehbar an einem distalen Endabschnitt des Handgriffabschnitts (4) gehalten ist und mit dem Endeffektor (8, 22) derart koppelbar ist, um eine Drehbewegung des Bedienelements (12) in einer ersten Drehrichtung in eine Bewegung des Endeffektors (8, 22) zu transformieren oder eine Funktion am Endeffektor (8, 22) zu bewirken. Dabei ist ein Winkelstellring (112), an einem distalen Ende des medizinischen Motorhandstücks (2) drehfest angeordnet und ist derart ausgebildet und vorgesehen, Winkelstellungen des Bedienelements (12) verrastend festzulegen. Die Offenbarung betrifft weiterhin ein medizinisches Handinstrument mit einem erfindungsgemäßen medizinischen Motorhandstück (2).

## Beschreibung

Die Offenbarung betrifft ein medizinisches Handstück mit einem Winkelstellring sowie ein medizinisches Handinstrument mit dem medizinischen Handstück.

### Hintergrund der Offenbarung

In der modernen minimalinvasiven Chirurgie werden derartige Werkzeuge/Instrumente und dazugehörige Instrumentenhandstücke/Handinstrumente beispielsweise zur Bearbeitung von Knochen, Knorpeln bei arthroskopischen Eingriffen, in der Wirbelsäulenchirurgie und dergleichen orthopädischen/chirurgischen Behandlungen sowie zur Bearbeitung von organischem Material in der Neurochirurgie verwendet. Die Werkzeuge/Instrumente weisen ein(en) Handstück/Handgriff/Griffabschnitt und ggf. austauschbare Effektoren, wie beispielsweise Fräser, Drehmesser, einen Polierkopf oder Ähnliches auf. Der Effektor ist in einem Schaft des Werkzeugs/Instruments an dessen distalem Ende gelagert, ggf. drehbar angetrieben gelagert. Als Werkzeugantrieb ist je nach Verwendungszweck und beabsichtigter Werkzeugdrehzahl ein hydraulischer, pneumatischer, oder elektromotorischer Antrieb vorgesehen, der über einen Drehmoment-Übertragungszug innerhalb des Werkzeugs und/ oder des Handinstruments bzw. des Griffabschnitts mit dem Werkzeugkopf (Effektor) wirkverbunden ist. Die Antriebe können dabei im Werkzeug und/oder im Handinstrument integriert oder als externe Antriebseinheiten ausgebildet sein, die über Energieversorgungsleitungen oder Drehmoment-Übertragungsstränge mit dem Werkzeug oder dem Handinstrument gekuppelt sind.

Dabei ist es vorteilhaft, den distalen Schaftabschnitt eines Schafts von einem medizinischen Handinstrument abzuwinkeln, um so Operationen in geringem Raum durchführen zu können, beispielsweise bei Operationen an der Wirbelsäule. Anders ausgedrückt, spielt bei chirurgischen, insbesondere minimal-invasiven, Eingriffen der Bauraum der verwendeten Instrumente und eine gute Handhabbarkeit eine große Rolle. So sollen die Instrumentenschäfte insbesondere im Bereich der distalen Effektoren auf möglichst kleinem Raum aktiv (über einen Betätigungsmechanismus gewollt ausgeführt) abwinkelbar sein.

Hierbei ist es von großer Bedeutung, dass der Betätigungsmechanismus durch einen Bediener intuitiv bedienbar ist. Es ist von Vorteil, wenn die verschiedenen Stellungen eines Bedienelements eines solchen Betätigungsmechanismus klar definiert und für den Bediener leicht erkennbar gestaltet sind.

### Stand der Technik

Abwinkelbare Schäfte für medizinische Handinstrumente sind hinlänglich bekannt und weisen üblicherweise einen proximalen und einen abwinkelbaren distalen Schaftabschnitt auf. Der distale Schaftabschnitt und der proximale Schaftabschnitt weisen dabei beide jeweils ein schräges Ende/ eine bezüglich der jeweiligen Schaftabschnittsachse angestellte Stirnseite auf. D.h. jeweils ein Ende/ Endabschnitt/ Stirnseite des distalen und proximalen Schaftabschnitts ist nicht gerade, sondern abgeschrägt/ angestellt. Die Schrägungen/ angestellten Endabschnitte/ Stirnseiten haben jeweils den im Wesentlichen gleichen Anstellwinkel. Darum passen die Schrägungen derart zueinander, dass der proximale und der distale Schaftabschnitt in einer bestimmten Relativdrehposition einen geraden Schaft/ ein gerades Rohr bilden. Wenn nun der distale Schaftabschnitt um seine Längsachse relativ zum proximalen Schaftabschnitt rotiert und der proximale Schaftabschnitt stehen bleibt, wird der distale Schaftabschnitt durch die angestellten Stirnseiten/ Endabschnitte zwangsläufig abgewinkelt.

Beispielsweise offenbart die DE 10 2017 010 033 A1 eine medizinische Vorrichtung mit einer Führungseinheit, die ein Führungsrohr mit einer Längsachse, ein fest mit diesem verbundenes proximales erstes Kopplungsteil und distal einen zylindermantelförmigen Schwenkkopf aufweist, sowie mit einem im Führungsrohr axial beweglichen, mit dem Schwenkkopf verbundenen Betätigungsrohr, das durch ein proximales Bedienungselement ein Verschwenken des Schwenkkopfes bewirkt. Zur präziseren Ausrichtung eines distalen Führungselements für ein drehbares chirurgisches Werkzeug und damit der winkelmäßigen Ausrichtung des Arbeitskopfes eines solchen Werkzeugs ist das Bedienungselement um die Längsachse verschwenkbar und bewirkt unter Axialverschiebung des Betätigungsrohrs das Verschwenken des Schwenkkopfes. Das Bedienelement ist mit einer über eine Feder vorgespannten Kugel ausgebildet, welche in Positionen bestimmter Winkelstellungen einrasten kann.

Des Weiteren sind in US 7,585,300 B2 oder US 10,070,872 B2 Beispiele für chirurgische Instrumente mit einem Handstück und einem in dem Handstück aufgenommenen Schaft, an dessen distalen Ende ein Werkzeugkopf schwenkbar angelenkt ist, offenbart. Die Verschwenkung des Werkzeugkopfs kann dabei über ein an dem Handstück drehbar angeordnetes Handrad bewirkt werden. Ähnlich dazu, ist in US 8,303,594 B2 ein chirurgisches Handstück offenbart, bei welchem die Verschwenkung des Werkzeugkopfs über einen an dem Handstück angeordneten Hebel bewirkt wird.

Darüber hinaus zeigt US 9,597,093 B2 ein Werkzeug, welches mit seinem proximalen Ende drehmomentübertragend mit einer Antriebseinheit gekoppelt werden kann und an seinem distalen Ende einen Werkzeugkopf aufweist. Der Werkzeugkopf kann durch Rotation einer in dem Bereich des Werkzeugkopfs angeordneten Hülse relativ zu einem Werkzeugschaft verschwenkt werden.

Aus dem Dokument US 2020/0170701 A1 ist ein Handstück mit einem Bedienelement zur Betätigung verschiedener Endeffektoren bekannt, die jedoch allesamt nicht-drehend sind. Das Bedienelement ist als Stellring ausgebildet, der um eine Achse drehbar gehalten ist, welche im Winkel zur Achse des Handgriffs steht.

Weitere Beispiele für chirurgische Handinstrumente mit rotierenden Werkzeugen, welche relativ zu einem Handstück angewinkelt / verschwenkt werden können, sind unter anderem in US 10,178,998 B2, US 10,307,180 B2 oder US 10,524,820 B2 offenbart.

Der Stand der Technik hat jedoch immer den Nachteil, dass das Bedienelement entweder keine Raststufen für verschiedene Winkelstellungen eines Betätigungsmechanismus aufweist oder, derart ausgebildet ist, dass die Montage eines entsprechenden Verrastungselements mit Raststufen aufwändig gestaltet ist. Insbesondere, erfordern derartige Ausgestaltungen bei einem Verschleiß solcher Raststufen einen Austausch des gesamten Verrastungselements bzw. des -systems.

### Zusammenfassung der Offenbarung

Es sind die Aufgaben und Ziele der Offenbarung, die Nachteile aus dem Stand der Technik zu beheben oder wenigstens zu mindern und insbesondere ein intuitives Motorhandstück bzw. Handinstrument bereitzustellen, welches ein Verrasten des Bedienelements einfach ausführt und die (De-)Montage eines entsprechenden Verrastungselements erleichtert.

Die Aufgaben und Ziele werden hinsichtlich eines gattungsgemäßen medizinischen Motorhandstücks offenbarungsgemäß durch den Gegenstand des Anspruchs 1 sowie eines gattungsgemäßen medizinischen Handinstruments mit dem medizinischen Motorhandstück offenbarungsgemäß durch den Gegenstand des Anspruchs 12 gelöst.

Die Offenbarung betrifft demzufolge ein medizinisches Motorhandstück zum Antreiben eines distalen Endeffektors, mit einem Handgriffabschnitt und einem vorzugsweise hülsenförmigen Bedienelement, das um eine Handgrifflängsachse drehbar an einem distalen Endabschnitt des Handgriffabschnitts gehalten / befestigt ist und mit dem Endeffektor derart koppelbar oder gekoppelt ist, um eine Drehbewegung des Bedienelements vorzugsweise in einer ersten Drehrichtung in eine Bewegung des Endeffektors zu transformieren und bei einer Drehbewegung des Bedienelements vorzugsweise in einer der ersten Drehrichtung entgegengesetzten, zweiten Drehrichtung eine Funktion am Endeffektor zu bewirken. Erfindungsgemäß weist das medizinische Motorhandstück an seinem distalen Ende bzw. Endbereich (distal zum Bedienelement) einen drehfesten Winkelstellring auf, welcher derart ausgebildet und vorgesehen ist, Winkelstellungen des Bedienelements verrastend festzulegen bzw. vorzugeben.

Anders ausgedrückt, ist ein Winkelstellring an einem distalen Ende/Endbereich des Motorhandstücks, insbesondere an diesem angrenzend, angeordnet und gibt festgelegte Winkelstellungen (mit erhöhtem Drehwiderstand) vor, in welche das Bedienelement des Motorhandstücks drehbar und verrastbar ist. Eine Drehung des Bedienelements erfolgt dabei schrittweise und der Winkelstellring gibt sozusagen die, vorzugsweise gleichmäßig aufgeteilten, Abstände dieser (Dreh-)Schritte bzw. die Abstände zwischen den jeweiligen Verrastzuständen des Motorhandstücks vor.

Durch die Anordnung eines solchen Winkelstellrings sind die Drehbewegungen des Bedienelements klar definiert / vorgegeben. Die Bewegungen bzw. Stellungen des Endeffektors sind somit ebenfalls festgelegt. Dem Bediener wird dadurch eine mühsame Feinjustierung erspart. Eine Anordnung des Winkelstellrings am distalen Ende des Motorhandstücks stellt ferner einen guten Zugriff auf diesen sicher und erleichtert dadurch eine (De-)Montage dieses Winkelstellrings, beispielsweise für Wartungstätigkeiten bzw. zum Auswechseln des Winkelstellrings.

Vorteilhafte Ausführungsformen sind in den Unteransprüchen beansprucht und werden nachfolgend erläutert.

Gemäß einem bevorzugten Aspekt der Offenbarung ist eine (äußere) Mantelfläche/Mantelseite des Winkelstellrings als eine Indikatorhülse / Markierungsring mit Winkelmarkierungen ausgebildet.

Anders ausgedrückt, weist der Winkelstellring eine (äußere) Indikatorhülse / Außenhülse auf, auf deren (äußere) Mantelfläche Winkelmarkierungen, vorzugsweise in Form von Winkel-Gradzahlen, vorgesehen/ausgebildet sind. Diese geben die verschiedenen Winkelstellungen / Drehstellungen des angrenzenden Bedienelements an, welches durch einen Verrastungsvorgang in den jeweiligen Stellungen gehalten ist. Dadurch wird dem Bediener deutlich angezeigt, in welchen Drehstellungen das Bedienelement aktuierbar ist bzw. in welcher Drehstellung sich das Bedienelement derzeitig befindet. Somit erfolgt die Bedienung / Aktuierung des Motorhandstücks klar und intuitiv.

Gemäß einem bevorzugten Aspekt der Offenbarung weist der Winkelstellring des medizinischen Motorhandstücks einen (inneren) Rastring auf, welcher in die Indikatorhülse einlegbar oder eingelegt ist und welcher eine Vielzahl an Ausnehmungen aufweist, die dafür vorgesehen sind, komplementäre Elemente des Bedienelements in den jeweiligen Winkelstellungen verrastend aufzunehmen.

In anderen Worten, ist ein Rastring / Rastblech im Inneren der Indikatorhülse einlegbar / anordenbar. Der Rastring weist eine Vielzahl an umfangsmäßig verteilten Ausnehmungen / Nuten auf, welche auf einer, vorzugsweise dem Bedienelement zugewandten Seite des Rastrings angeordnet sind und der Verrastung von komplementären Elementen, vorzugsweise in Form eines Zapfenabschnitts oder in Form von Kugeldruckelementen, des Bedienelements dienen.

Dadurch, dass die Ausnehmungen zur Verrastung bzw. zur Aufnahme der komplementären Elemente des Bedienelements an einem separaten Rastring ausgebildet sind und dieser in den Winkelstellring (drehfest) einlegbar ist, muss bei einem eventuellen Verschleiß nur dieser Rastring ausgewechselt werden, wodurch der Einsatz eines solchen Winkelstellrings preisgünstig und einfach gestaltbar ist.

Gemäß einem bevorzugten Aspekt der Offenbarung weist der Rastring des Winkelstellrings ein Übermaß bezogen auf dessen Durchmesser auf, sodass der Rastring beim Einlegen in die Indikatorhülse des Winkelstellrings selbsttätig in der Indikatorhülse fixierbar oder fixiert ist.

In anderen Worten, ist der (Außen-)Durchmesser des Rastringes etwas größer als der Innendurchmesser der Indikatorhülse ausgebildet, sodass dieser in diesem werkzeuglos fixierbar (ausschließlich pressgepasst) ist. Eine Demontage des Rastringes erfolgt dann beispielsweise durch eine einfache Hebelbewegung. Dies vereinfacht die (De-)Installation des Rastringes innerhalb des Winkelstellrings.

In einem weiteren bevorzugten Aspekt der Offenbarung weist der Rastring eine konische Mantelfläche auf.

In einem weiteren bevorzugten Aspekt der Offenbarung ist die Fläche der Indikatorhülse, an welche die Mantelfläche des eingelegten Rastringes angrenzt, konisch ausgebildet.

Eine derartige konische Ausbildung der Kontaktflächen des Rastringes bzw. der Indikatorhülse, welche in einem eingelegten Zustand des Rastringes einander angrenzen, bewirkt, insbesondere in Kombination eines Übermaßes des Rastringes, eine effektive axiale Sicherung des Rastringes innerhalb der Indikatorhülse. Vorzugsweise ist sind diese konischen Flächen um etwa 2-3° geneigt ausgebildet.

In einem weiteren bevorzugten Aspekt der Offenbarung weist der Winkelstellring einen innenliegenden, von außen nicht sichtbaren Sprengring auf, mithilfe dessen der Winkelstellring an einem Schaft des Motorhandstücks axialgesichert befestigbar oder befestigt ist.

Anders ausgedrückt, ist innerhalb der äußeren Indikatorhülse ein Sprengring angeordnet, welcher von der Indikatorhülse von außen abgedeckt wird, wodurch dieser von außen nicht sichtbar ist. Der Sprengring ist derart ausgebildet, dass dieser um den Schaft des Motorhandstücks fixierbar ist bzw. sich um diesen klemmt. Vorzugsweise erfolgt dieses Klemmen des Sprengrings innerhalb einer radial umlaufenden Außennut am Schaft des Motorhandstücks. Somit erfolgt die Fixierung des gesamten Winkelstellrings durch den in diesen integrierten Sprengring auf dem Trägerbauteil / Schaft des Motorhandstücks.

Dies ermöglicht einen axial besonders kurz ausgebildeten Markierungs- und Fixierungsring am Motorhandstück, wodurch der Winkelstellring kompakt ausbildbar ist. Des Weiteren, ist eine Demontage des von außen unsichtbaren Sprengrings und somit des gesamten Winkelstellrings nur durch den Einsatz eines Spezialwerkzeugs möglich, wodurch insbesondere ein ungewolltes Lösen des Winkelstellrings erschwert ist.

Gemäß einem weiteren bevorzugten Aspekt der Offenbarung ist der Rastring des Winkelstellrings aus einem gehärteten Stahl gefertigt.

Dadurch, dass der Rastring separat ausgebildet ist und vorzugsweise aus einem härteren (Stahl-)Material als die restlichen / umliegenden Komponenten gefertigt ist, wird ein Verschleiß des Rastringes durch die immer wieder auftretenden Verrastungsvorgänge erschwert. Die Notwendigkeit den Rastring des Winkelstellrings auszutauschen / zu ersetzen ist somit geringgehalten.

Gemäß einer vorteilhaften Weiterbildung ist die äußere Mantelfläche der Indikatorhülse konisch ausgebildet und verengt sich im Durchmesser zum distalen Ende des Motorhandstücks hin. Dadurch wird eine ergonomische Handhabung des Handstücks ermöglicht.

Die Offenbarung betrifft weiterhin ein medizinisches Handinstrument mit einem offenbarungsgemäßen, medizinischen Motorhandstück und einem Endeffektor, welcher über einen Schaft mit dem Motorhandstück gekoppelt ist, um ein Drehmoment von dem Motorhandstück auf den Endeffektor zu übertragen.

### Kurzbeschreibung der Figuren

Die Offenbarung wird nachfolgend anhand bevorzugter Ausführungsbeispiele mit Hilfe von Figuren näher erläutert. Es zeigen:
- Fig. 1: eine perspektivische Ansicht eines medizinischen Handinstruments gemäß einer ersten Ausführungsform der vorliegenden Offenbarung;
- Fig. 2: eine weitere perspektivische Ansicht des medizinischen Handinstruments gemäß der ersten Ausführungsform der vorliegenden Offenbarung;
- Fig. 3: eine Teillängsschnittansicht eines Motorhandstücks des medizinischen Handinstruments gemäß der ersten Ausführungsform;
- Fig. 4: eine Querschnittansicht des Motorhandstücks des medizinischen Handinstruments gemäß der ersten Ausführungsform;
- Fig. 5: eine Teillängsschnittansicht eines distalen Endabschnitts des medizinischen Handinstruments gemäß der ersten Ausführungsform in einer geraden Schaftform;
- Fig. 6: eine Teillängsschnittansicht des distalen Endabschnitts des medizinischen Handinstruments gemäß der ersten Ausführungsform in einer abgewinkelten Schaftform;
- Fig. 7: eine Teillängsschnittansicht des distalen Endabschnitts des medizinischen Handinstruments gemäß der ersten Ausführungsform in einem Freigabezustand;
- Fig. 8: eine isometrische Perspektivansicht des Motorhandstücks des medizinischen Handinstruments gemäß der ersten Ausführungsform;
- Fig. 9: eine Teillängsschnittansicht des Motorhandstücks des medizinischen Handinstruments gemäß der ersten Ausführungsform;
- Fig. 10: eine perspektivische Längsschnittansicht des medizinischen Handinstruments gemäß einer Modifikation der ersten Ausführungsform;
- Fig. 11: eine Detailansicht einer Abgleitkulisse des medizinischen Handinstruments gemäß der Modifikation der ersten Ausführungsform;
- Fig. 12: eine perspektivische Ansicht des medizinischen Handinstruments gemäß der ersten Ausführungsform in einem Betriebszustand;
- Fig. 13: eine perspektivische Ansicht des medizinischen Handinstruments gemäß der ersten Ausführungsform in einem Betriebszustand;
- Fig. 14: eine isometrische Perspektivansicht eines Motorhandstücks eines medizinischen Handinstruments gemäß einer zweiten Ausführungsform;
- Fig. 15: eine Längsschnittansicht des medizinischen Handinstruments gemäß der zweiten Ausführungsform; und
- Fig. 16: eine Querschnittansicht des medizinischen Handinstruments gemäß der zweiten Ausführungsform.
- Fig. 17: eine weitere Querschnittansicht des Motorhandstücks des medizinischen Handinstruments gemäß der ersten Ausführungsform;
- Fig. 18: eine isometrische Explosionsdarstellung des Winkelstellrings des Motorhandstücks;
- Fig. 19: eine isometrische Perspektivansicht der Rückseite des Winkelstellrings in einem zusammengesetzten Zustand; und
- Fig. 20: eine Teillängsschnittansicht des Motorhandstücks mit dem montierten Winkelstellring, gemäß der ersten Ausführungsform.

Die Figuren sind schematischer Natur und dienen lediglich dem Verständnis der Offenbarung. Gleiche Elemente sind mit denselben Bezugszeichen versehen. Die Merkmale der verschiedenen Ausführungsbeispiele können untereinander ausgetauscht werden.

### Detaillierte Beschreibung der Figuren

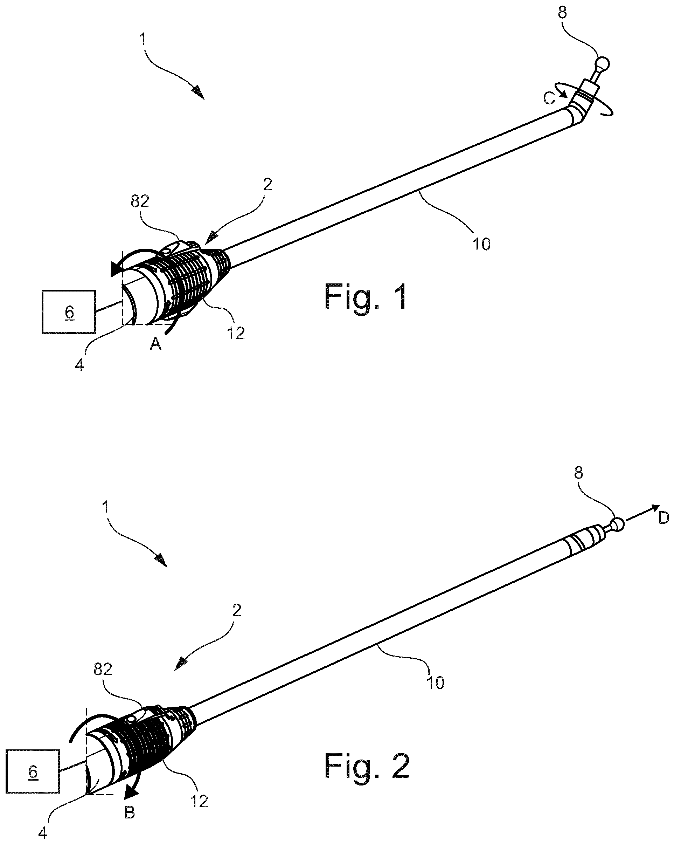

Figur 1 zeigt perspektivisch ein medizinisches Handinstrument 1 gemäß einer ersten Ausführungsform der vorliegenden Offenbarung. Das medizinische Handinstrument 1 weist dabei ein medizinisches Motorhandstück 2 auf, welches einen proximalen Handgriffabschnitt 4 aufweist und welches, wie in Fig. 1 schematisch dargestellt, mit einer Antriebseinheit 6 gekoppelt werden kann. Alternativ kann die Antriebseinheit 6 auch in dem Motorhandstück 2 aufgenommen sein und über einen Energieversorgungsanschluss mit Energie versorgt werden.

Ferner weist das Handinstrument 1 ein distales Werkzeug (Endeffektor/Effektorabschnitt) 8 auf, welches über einen (Werkzeug-)Schaft 10 mit dem Motorhandstück 2, insbesondere mit dem Handgriffabschnitt 4, gekoppelt werden kann oder von diesem entkoppelt werden kann. Das Werkzeug 8 kann dabei beispielsweise als Fräser, Bohrer oder Polierkopf ausgeführt sein.

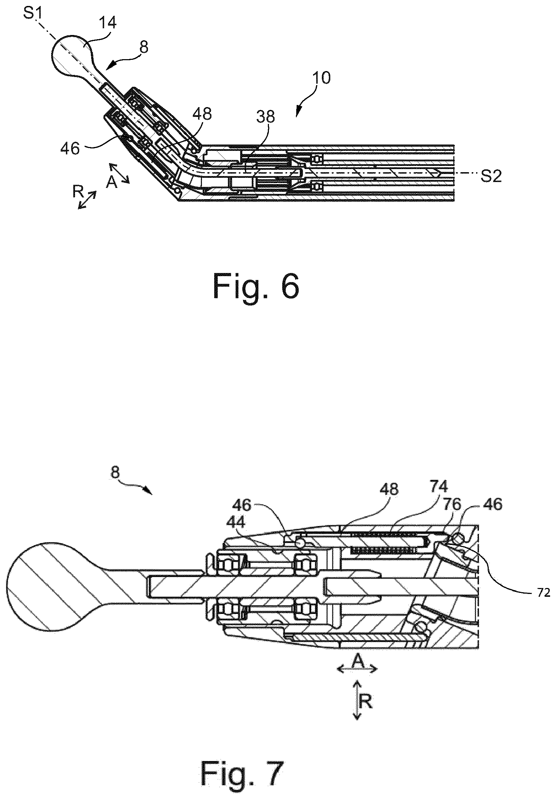

Wenn der Schaft 10 mit dem Handgriffabschnitt 4 gekoppelt ist, wird Drehmoment von der Antriebseinheit 6 über einen in dem Handinstrument 1, insbesondere dem Handgriffabschnitt 4 und dem Schaft 10, angeordneten Drehmoment-Übertragungszug hin zu dem Werkzeug 8 übertragen, um dieses in Drehung zu versetzen. Bei Verwendung des Handinstruments 1 im Rahmen einer chirurgischen Operation bzw. medizinischen Behandlung ist es bekannt, dass das Werkzeug 8 relativ zu dem Schaft 10 abgewinkelt werden kann (in Fig. 1 durch den Pfeil C angedeutet). D.h. das Werkzeug 8 und der Schaft 10 können, wie nachstehend näher beschrieben, so zueinander abgewinkelt werden, dass eine Längsachse S1 des Werkzeugs 8 und eine Schaftlängsachse S2 des Schafts 10 miteinander einen Winkel ungleich 180°, insbesondere kleiner als 180°, ausbilden (vgl. Fig. 6).

Wie in Fig. 1 gezeigt, ist hierfür bei dem offenbarungsgemäßen Handinstrument 1 ein hülsenförmiges Bedienelement 12 vorgesehen. Das Bedienelement 12 ist dabei an einem distalen Endabschnitt des Handgriffabschnitts 4 angeordnet und um eine Handgrifflängsachse in einer ersten Drehrichtung A und in einer der ersten Drehrichtung A entgegengesetzten, zweiten Drehrichtung B drehbar. Mit anderen Worten ausgedrückt, weist das Motorhandstück 2 den Handgriffabschnitt 4 und das distal angeordnete Bedienelement 12 auf, welche koaxial zueinander angeordnet sind, wobei das Bedienelement 12 relativ zu dem Handgriffabschnitt 4 gedreht werden kann.

Eine Drehung des Bedienelements 12 aus einer neutralen Nullstellung, bei welcher das Werkzeug 8 nicht relativ zu dem Schaft 10 abgewinkelt ist, in der ersten Drehrichtung A bewirkt dabei, wie in Fig. 1 gezeigt, ein Abwinkeln des Werkzeugs 8 relativ zu dem Schaft 10. Die erste Drehrichtung A ist bei dem Handinstrument 1 gemäß der vorliegenden Offenbarung als eine Drehung des Bedienelements 12 relativ zu dem Handgriffabschnitt 4 nach links definiert. Anders ausgedrückt entspricht die erste Drehrichtung A in einer distalen Draufsicht auf das Werkzeug 8 einer Drehung des Bedienelements 12 im Uhrzeigersinn.

Wie in Fig. 2 gezeigt, kann das Bedienelement 12 bei dem offenbarungsgemäßen Handinstrument 1 auch in der zweiten Drehrichtung B, welche der ersten Drehrichtung A entgegengesetzt ist, relativ zu dem Handgriffabschnitt 4 gedreht werden. D.h. die zweite Drehrichtung B ist in der Draufsicht auf das Werkzeug 8 als eine Drehung des Bedienelements 12 entgegen dem Uhrzeigersinn bzw. als eine Drehung nach rechts definiert. Wie durch den Pfeil D in Fig. 2 angedeutet, bewirkt eine Drehung des Bedienelements 12 in der zweiten Drehrichtung B ein Lösen der Kopplung des Werkzeugs 8 mit dem Schaft 10. Somit kann, wie nachstehend näher erläutert, durch eine Drehung des Bedienelements 12 in der zweiten Drehrichtung B ein einfacher und schneller Werkzeugwechsel gewährleistet werden.

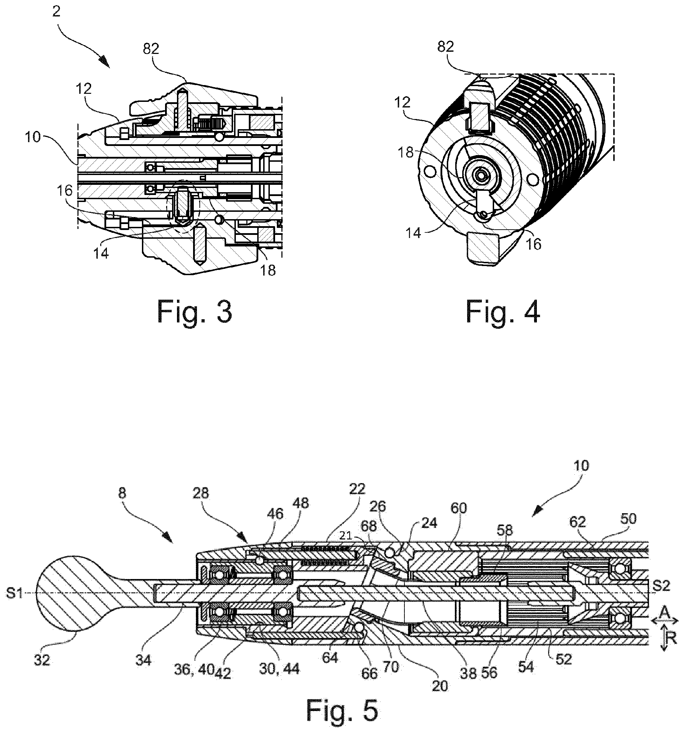

Fign. 3 und 4 zeigen Teilschnittansichten des Handinstruments 1 gemäß der ersten Ausführungsform. Zur Übertragung der Drehbewegung des Bedienelements 12 auf den Schaft 10 ist in radialer Richtung des Bedienelements 12 ein Verstellstift 14 angeordnet. Ein radial außenliegender Endabschnitt des Verstellstifts 14 ist dabei in einer Axialnut 16, welche auf einer Innenumfangsfläche des Bedienelements 12 ausgebildet ist, aufgenommen. Auf einer radialen Innenseite ist der Verstellstift 14 in einer Drehübertragungshülse 18 aufgenommen. Die Drehübertragungshülse 18 ist dabei drehfest mit dem Schaft 10 verbunden, so dass eine Bewegung des Verstellstifts 14 in Umfangsrichtung eine Drehung der Drehübertragungshülse 18 und des Schafts 10 bewirkt. Anders ausgedrückt, bewegt sich bei Verdrehung des Bedienelements 12 in der ersten Drehrichtung A oder in der zweiten Drehrichtung B der Verstellstift 14 in der Axialnut 16 mit dem Schaft 10 mit und bewirkt je nach Drehrichtung das Abwinkeln bzw. den Auswurf des Werkzeugs 8.

Fig. 5 zeigt einen Längsquerschnitt eines distalen Endabschnitts des Handinstruments 1 in einer geraden Schaftform. D.h. das Bedienelement 12 ist nicht relativ zu dem Handgriffabschnitt 4 verdreht (Nullstellung). Demzufolge sind das Werkzeug 8 und der Schaft 10 nicht abgewinkelt und die Längsachse S1 des Werkzeugs 8 ist kollinear zu der Schaftlängsachse S2. Wie in Fig. 5 zu erkennen, weist der Schaft 10 einen, dem Handgriffabschnitt 4 zugewandten und mit diesem koppelbaren, proximalen Schaftabschnitt 20 und einen mit dem Werkzeug 8 koppelbaren distalen Schaftabschnitt 22 auf. Wenn das Bedienelement 12 nicht relativ zu dem Handgriffabschnitt 4 verdreht ist und der Schaft 10 in der geraden Schaftform vorliegt, sind der proximale Schaftabschnitt 20 und der distale Schaftabschnitt 22 demzufolge in einer Linie angeordnet bzw. weisen einen Winkel von 0° zueinander auf. Der proximale Schaftabschnitt 20 ist dabei im Wesentlichen rohrförmig ausgestaltet und weist an seinem distalen Endabschnitt eine in Schaftlängsachse S2 angestellte Stirnseite 24 auf. Der distale Schaftabschnitt 22 ist ebenfalls annähernd rohrförmig ausgeführt. Das Rohr läuft zum distalen Ende hin spitzförmig zu. Der distale Schaftabschnitt 22 weist an seinem proximalen Endabschnitt eine in Schaftlängsachse S2 angestellte Stirnseite 26 auf.

Die angestellten Stirnseiten 24, 26 weisen jeweils einen Anstellwinkel von vorzugsweise 22,5° zu einer Normalenebene zur Schaftlängsachse S2 auf. Wenn der Schaft 10 in einer geraden Schaftform bzw. ausgestreckt ist, sind die beiden angestellten Stirnseiten 24, 26 derart zueinander versetzt, dass sich die langen Enden der angestellten Stirnseiten 24, 26 in Relation zur Schaftlängsachse S2 gegenüberliegen. Die angestellten Stirnseiten 24, 26 liegen aufeinander auf. Die angestellten Stirnseiten 24, 26 müssen dabei nicht zwangsläufig einen Anstellwinkel von 22,5° aufweisen. Es sind auch Anstellwinkel von beispielsweise 10°, 18°, 30°, 45° oder jeder andere Anstellwinkel denkbar.

Wie vorstehend erwähnt, ist der distale Schaftabschnitt 22 ausgebildet, mit dem Werkzeug 8 gekoppelt zu werden. D.h. der distale Schaftabschnitt 22 stellt eine Werkzeugaufnahme dar, wobei das Werkzeug 8 in der Werkzeugaufnahme aufgenommen ist und relativ zu der Werkzeugaufnahme, d.h. dem distalen Schaftabschnitt 22, drehbar gelagert ist.

Hierfür ist in dem distalen Schaftabschnitt 22 eine erste Kupplungsvorrichtung 28 angeordnet. An dem Werkzeug 8 ist eine zweite Kupplungsvorrichtung 30 vorgesehen, die in einem Kupplungszustand durch Zusammenwirken mit der ersten Kupplungsvorrichtung 28 eine Kupplung zwischen dem Werkzeug 8 und dem distalen Schaftabschnitt 22 realisiert, um das Werkzeug 8 im distalen Schaftabschnitt 22 in Axialrichtung A zu fixieren.

Wie vorstehend erwähnt, schließen die Längsachse S1 des Werkzeugs 8 bzw. des distalen Schaftabschnitts 22 und die Schaftlängsachse S2, d.h. die Längsachse des proximalen Schaftabschnitts 20 in Fig. 5 einen Winkel von 0° ein. Zudem ist in Fig. 5 ein Kupplungszustand zwischen dem Werkzeug 8 und dem distalen Schaftabschnitt 22 gezeigt. Das heißt, dass die erste Kupplungsvorrichtung 28, die im/am distalen Schaftabschnitt 22 angebracht ist, und die zweite Kupplungsvorrichtung 30, die am Werkzeug 8 vorgesehen ist, miteinander in Eingriff stehen bzw. zusammenwirken. Das Werkzeug 8 weist einen Werkzeugkopf/Effektor 32, beispielsweise einen Fräskopf, und einen Werkzeugschaft 34 auf. Der Werkzeugkopf 32 und der Werkzeugschaft 34 sind drehfest miteinander verbunden.

In Fig. 5 ist weiterhin zu erkennen, dass der Werkzeugschaft 34 mithilfe einer Wälzlagereinheit 36 in dem distalen Schaftabschnitt 22 drehbar gelagert ist. Durch den proximalen Schaftabschnitt 20 erstreckt sich eine Antriebswelle 38, welche drehfest mit dem Werkzeugschaft 34 verbunden ist und in Form eines Drehmomentübertragungszugs Drehmoment von der Antriebseinheit 6 auf den Werkzeugschaft 34 aufbringt. Der Werkzeugschaft 34 dreht sich infolge dieser Drehmomentbeaufschlagung relativ zu dem distalen Schaftabschnitt 22. Die Wälzlagereinheit 36 weist zumindest ein, hier genau zwei, voneinander in Axialrichtung A beabstandete Wälzlager 40, genauer gesagt Kugellager, auf, die alternativ aber auch als Gleitlager ausgebildet sein können. Die Wälzlager 40 sind in einem Lagergehäuse 42, das Teil der Wälzlagereinheit 36 ist, aufgenommen.

Die zweite Kupplungsvorrichtung 30 ist in das Lagergehäuse 42 eingebracht und damit nicht direkt an dem Werkzeug 8 vorgesehen. Die Wälzlagereinheit 36 und damit auch das Lagergehäuse 42 ist an dem Werkzeugschaft 34 in Axialrichtung A fixiert angeordnet. Alternativ dazu wäre es aber auch denkbar, dass die zweite Kupplungsvorrichtung 30 direkt an dem Werkzeugschaft 34 vorgesehen ist. Die zweite Kupplungsvorrichtung 30 ist als eine sich in Umfangsrichtung des Lagergehäuses 42 durchgängig ersteckende bzw. umlaufende Axialsicherungsnut 44 ausgebildet. Die Axialsicherungsnut 44 hat vorzugsweise einen halbkreisförmigen oder kreissegmentförmigen Querschnitt.

Die erste Kupplungsvorrichtung 28, die in dem distalen Schaftabschnitt 22 vorgesehen ist, weist zumindest eine Verriegelungskugel 46 auf. Der Durchmesser der Verriegelungskugel 46 ist so gewählt, dass die Verriegelungskugel 46 in der Axialsicherungsnut 44 aufnehmbar ist. Vorzugsweise umgibt die Axialsicherungsnut 44 zumindest einen die Axialsicherungsnut 44 kontaktierenden Abschnitt der Verriegelungskugel 46 vollumfänglich. Die Verriegelungskugel 46 wird im Kupplungszustand an ihrer der Axialsicherungsnut 44 gegenüberliegenden Seite von einem distalen Ende eines Raststiftes/Schiebers 48 in der Axialsicherungsnut 44 gehalten. Der Raststift 48 ist ein Teil der ersten Kupplungsvorrichtung 28. Der Raststift 48 ist in Radialrichtung R fixiert. Der Raststift 48 ist in Axialrichtung verschieblich bzw. beweglich in dem distalen Schaftabschnitt 22 angeordnet.

In dem in Fig. 5 gezeigten Kupplungszustand ist also die Verriegelungskugel 46 in der Axialsicherungsnut 44 aufgenommen und wird von dem Raststift 48 in Radialrichtung in der Axialsicherungsnut 44 gehalten. Diese Position des Raststiftes 48 in Axialrichtung A wird als Kupplungsposition bezeichnet. Eine Kante 21 am distalen Ende des proximalen Schaftabschnittes 20 verhindert eine Bewegung des Raststiftes 48 in Axialrichtung hin zu dem proximalen Schaftabschnitt 20.

Der proximale Schaftabschnitt 20 weist ein feststehendes Außenrohr 50, ein Hohlrad 52 mit einer Innenverzahnung 54, ein Ritzel 56 mit einer Außenverzahnung 58 und eine exzentrische Sicherungsbuchse 60 auf. Das Hohlrad 52 ist innerhalb des Außenrohrs 50 positioniert und die Längsachse des Außenrohrs 50 entspricht der Längsachse des Hohlrads 52. Das Außenrohr 50 und das Hohlrad 52 sind also konzentrisch angeordnet. Das Hohlrad 52 ist über eine in dem proximalen Schaftabschnitt 20 aufgenommene Hohlwelle 62 mit der Drehübertragungshülse 18, d.h. mit dem Bedienelement 12, verbunden.

Wie vorstehend erwähnt, ist das Außenrohr 50 als feststehendes Rohr ausgeführt und bewegt sich demnach nicht. Das distale Ende des Außenrohrs 50 weist die angestellte Stirnseite 24 auf. Das distale Ende des Außenrohrs 50 weist weiterhin eine Aufnahmebohrung 64 und einen Aufnahmezapfen für ein Wälzlager 66 auf. Das Außenrohr 50 weist eine Rille/ Nut für die Kugeln des Wälzlagers 66 auf. Auf dem Wälzlager 66 ist der distale Schaftabschnitt 22 gelagert.

Die Innenverzahnung 54 kämmt mit der Außenverzahnung 58 des Ritzels 56. Dadurch wird eine Rotation des Hohlrads 52, die durch das Bedienelement 12 gesteuert wird, auf das Ritzel 56 übertragen. Die Drehrichtung des Ritzels 56 ist dabei gleichgesetzt der Drehrichtung des Hohlrads 52. Das Ritzel 56 wird vom Hohlrad 52 angetrieben, das Ritzel 56 dreht sich jedoch in der exzentrischen Sicherungsbuchse 60. Die Sicherungsbuchse 60 ist exzentrisch zum Hohlrad 52 angeordnet. D.h. die Längsachse der exzentrischen Sicherungsbuchse 60 ist zwar parallel zur Längsachse des Hohlrads 52, die Längsachsen liegen aber nicht übereinander bzw. versetzt zueinander. Der distale Schaftabschnitt 22 weist eine Verstellbuchse 68 auf. Die Verstellbuchse 68 ist in der Aufnahmebohrung 64 des proximalen Schaftabschnitts 20 gelagert. Die Verstellbuchse 68 ist über einen flexiblen Silikonschlauch 70 mit dem Ritzel 56 derart verbunden, dass eine Rotation des Ritzels 56 auf die Verstellbuchse 68 übertragen wird. Hierfür ist der flexible Silikonschlauch 70 an der Verstellbuchse 68 und dem Ritzel 56 beispielsweise durch Schweißen oder Kleben befestigt. Die Verstellbuchse 68 ist ferner über einen Mitnahmezapfen (nicht dargestellt) mit dem distalen Schaftabschnitt 22 formschlüssig verbunden ist, so dass eine Rotation der Verstellbuchse 68 auf den distalen Schaftabschnitt 22 übertragen wird.

Fig. 6 zeigt einen Längsquerschnitt durch den distalen Endabschnitt des Schafts 10, wobei der distale Schaftabschnitt 22 zu dem proximalen Schaftabschnitt 20 bei einem jeweiligen Anstellwinkel der beiden Stirnseiten 24, 26 um 22,5° folglich um 45° abgewinkelt ist. Der distale Schaftabschnitt 22 ist im Vergleich zur Stellung in Fig. 5 um 180° um die eigene Längsachse S1 gedreht. Die angestellten Stirnseiten 24, 26 liegen in dieser Stellung wieder komplett / flächig aufeinander auf. Durch die Drehung des distalen Schaftabschnitts 22 sind die langen Enden der angestellten Stirnseiten 24, 26 aber nebeneinander positioniert. Die Anstellwinkel der angestellten Stirnseiten 24, 26 addieren sich somit. Dadurch wird der distale Schaftabschnitt 22 im Vergleich zum proximalen Schaftabschnitt 20 um den doppelten Anstellwinkel der angestellten Stirnseiten 24, 26 abgewinkelt.

In der in Fig. 6 gezeigten Stellung, ist der Mitnahmezapfen gegenüber der Sicherungsbuchse 60 angeordnet. D.h. die Verstellbuchse 68 hat sich von der ausgestreckten Position zur maximalen Abwinklung um 180° gedreht. Die 45°-Position stellt für diese Konstruktion den Umkehrpunkt dar. Die Verstellbuchse 68 hat sich in dieser Position um 180° gedreht. Bei weiterer Drehung des Hohlrads 52, d.h. des Bedienelements 12, würde sich der distale Schaftabschnitt 22 wieder in die Ausgangsstellung (Nullstellung) zurück drehen.

Ferner ist in Fig. 6 zu erkennen, dass die Verriegelungskugel 46 auch dann, wenn der distale Schaftabschnitt 22 in der maximal einstellbaren Winkelstellung bzw. maximale Abwinklung bezüglich des proximalen Schaftabschnitts 20 abgewinkelt ist, durch den Raststift 48 in Radialrichtung R in der Axialsicherungsnut 44 gehalten ist. Auf diese Weise ist das Werkzeug 8 in dem distalen Schaftabschnitt durch den Eingriff zwischen erster Kupplungsvorrichtung 28 und zweiter Kupplungsvorrichtung 30 auch in dieser Winkelstellung in Axialrichtung A gesichert.

Der Drehmomentstrang, insbesondere die Antriebswelle 38, hin zum Werkzeugschaft 34, der im Übergangsbereich zwischen dem distalen Schaftabschnitt 22 und dem proximalen Schaftabschnitt 20 angeordnet ist, ist flexibel. Dieser flexible Abschnitt des Drehmomentstrangs erlaubt, dass der distale Abschnitt des Werkzeugschaftes 34 mit Werkzeugkopf 32 zusammen mit dem distalen Schaftabschnitt 22 relativ zum Drehmomentstrang im proximalen Schaftabschnitt 20 abgewinkelt werden kann. Gleichzeitig ist der flexible Abschnitt des Drehmomentstrangs so ausgeführt, dass er ein Drehmoment, das auf einen proximalen Abschnitt des Werkzeugschaftes 34 ausgeübt wird, weiterhin auf den Werkzeugkopf 32 übertragen kann.

Fig. 7 ist eine Längsschnittansicht des distalen Endabschnitts des Schafts 10 in einem Freigabezustand zwischen dem Werkzeug 8 und dem distalen Schaftabschnitt 22. D.h. in der in Fig. 7 gezeigten Stellung stehen die die erste Kupplungsvorrichtung 28 und die zweite Kupplungsvorrichtung 30 nicht in Wirkeingriff miteinander, so dass das Werkzeug 8 entnommen bzw. gewechselt werden kann. Um den Wirkeingriff der ersten Kupplungsvorrichtung 28 und der zweiten Kupplungsvorrichtung 30 zu lösen, wird das Bedienelement 12 in der zweiten Drehrichtung B gedreht (vgl. Fig. 2). Wie nachstehend näher erläutert, ist die Verriegelungskugel 46 dabei nicht mehr in der Axialsicherungsnut 44 aufgenommen. Stattdessen ist sie von dem Raststift 48 in Radialrichtung R gegen eine Außenumfangsfläche des Lagergehäuses 42 gehalten.

Damit sich die Verriegelungskugel 46 ausgehend von dem, in Fig. 5 gezeigten Kupplungszustand, aus der Axialsicherungsnut 44 heraus in den Freigabezustand bewegen kann, muss der Raststift 48 sich in Axialrichtung A hin zu dem proximalen Schaftabschnitt 20 bewegen. Dies wird im Kupplungszustand durch die umlaufende Kante des proximalen Schaftabschnitts 20 verhindert. Die Kante ist jedoch an einer Stelle einer Raststift-Aufnahmevertiefung 72 unterbrochen. Wenn das Bedienelement 12 in der zweiten Drehrichtung B gedreht wird, rotiert der distale Schaftabschnitt 22 relativ zu dem proximalen Schaftabschnitt 20 in einer dem Abwinkeln entgegengesetzten Richtung, beispielsweise um (-)18°, bis der Raststift 48 relativ zu dem proximalen Schaftabschnitt 20 so positioniert ist, dass er in Umfangsrichtung auf einer Höhe mit der Raststift-Aufnahmevertiefung 72 ist. Ein Vorspannelement 74, das ein Teil der ersten Kupplungsvorrichtung 28 ist, drückt den Raststift 48 in Axialrichtung A zum proximalen Schaftabschnitt 20 hin. Somit schiebt das Vorspannelement 74 den Raststift 48, genauer gesagt dessen proximales Ende, das als Rastvorsprung 76 ausgebildet ist, in die Raststift-Aufnahmevertiefung 72 hinein. Die Position, in der der Raststift 48 ist, wenn sein Rastvorsprung 76 in die Raststift-Aufnahmevertiefung 72 eingreift, wird als der Freigabezustand bzw. Freigabeposition bezeichnet.

In der Freigabeposition liegt das distale Ende des Raststifts 48 nicht mehr der Axialsicherungsnut 44 gegenüber. Damit wird die Verriegelungskugel 46 in Radialrichtung R nicht in der Axialsicherungsnut 44 gehalten. Das Werkzeug 8 ist somit relativ zum distalen Schaftabschnitt 22 nicht mehr in Axialrichtung A fixiert. Wird nun, ausgehend vom Kupplungszustand, eine Zugkraft (in Axialrichtung A) auf das distale Ende des Werkzeugs 8 aufgebracht, löst sich die Verriegelungskugel 46 aus der Axialsicherungsnut 44. Auf diese Weise kann das Werkzeug 8 von dem distalen Schaftabschnitt 22 entkuppelt werden.

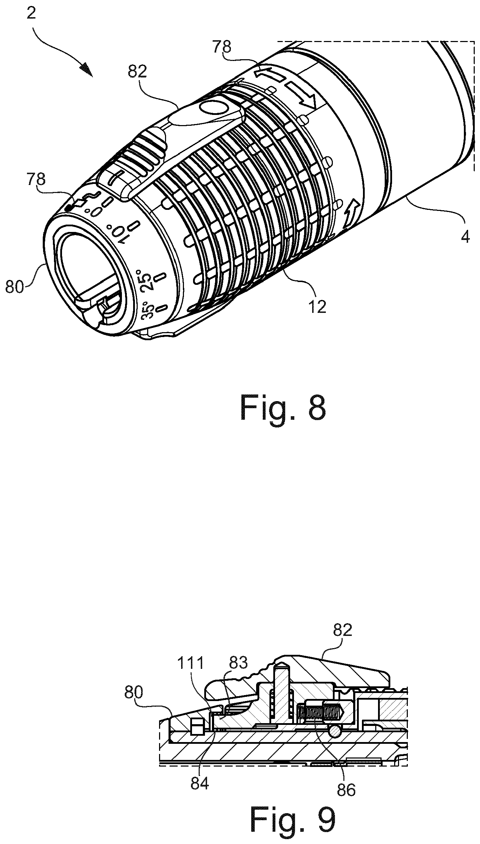

Fig. 8 zeigt eine Perspektivansicht des distalen Endabschnitts des Motorhandstücks 2 gemäß der ersten Ausführungsform, ohne dass der Schaft 10 mit dem Handgriffabschnitt 4 gekoppelt ist. Wie vorstehend beschrieben, bewirkt eine Drehung des Bedienelements 12 in der ersten Drehrichtung A ein Abwinkeln des Werkzeugs 8 und eine Drehung des Bedienelements 12 in der zweiten Drehrichtung B löst die Kupplung zwischen dem Werkzeug 8 und dem Schaft 10, bzw. dem distalen Schaftabschnitt 22, indem der Wirkeingriff zwischen der ersten Kupplungsvorrichtung 28 und der zweiten Kupplungsvorrichtung 30 gelöst wird. Zur Erleichterung der Bedienung sind an dem Bedienelement 12 Indikatoren 78 in Form von Pfeilen, welche die Drehrichtungen angeben, angebracht. In Kombination mit Indikatoren 78 auf einer Indikatorhülse 80, welche distal des Bedienelements 12 drehfest an dem Handgriffabschnitt 4 angebracht ist, kann der Verwender bei der Bedienung des Handinstruments 1 schnell erkennen, welche Drehrichtung welcher Funktion entspricht. Hierfür sind, wie in Fig. 8 gezeigt, auf einer Außenumfangsfläche der Indikatorhülse 80 verschiedene Winkelpositionen, welche jeweils einer definierten Drehung des Bedienelements 12 in der ersten Drehrichtung A und damit einem definierten Abwinkeln des Werkzeugs 8 relativ zu dem Schaft 10 entsprechen, sowie ein Schloss-Symbol, bzw. ein Symbol eines geöffneten Schlosses, aufgebracht. Der Verwender kann somit den einzelnen Drehrichtungen A, B die jeweilige Funktion, nämlich das Abwinkeln oder das Entkuppeln, zuordnen. Das Motorhandstück 2 mitsamt dem Bedienelement 12 ermöglicht somit eine intuitive Bedienung und Integration der beiden Funktionen.

Wie in Fig. 8 gezeigt, ist an dem Motorhandstück 2 gemäß der ersten Ausführungsform ein Verriegelungsschieber 82 angeordnet. Der Verriegelungsschieber 82 ist dabei in Radial- und in Umfangsrichtung fest so in dem Bedienelement 12 aufgenommen, dass sich der Verriegelungsschieber 82 lediglich in Axialrichtung relativ zu dem Bedienelement 12 zwischen einer (distalen) Verriegelungs- und einer (proximalen) Entriegelungsposition bewegen kann. In der Verriegelungsposition kann das Bedienelement 12, wie nachstehend näher erläutert, nicht relativ zu dem Handgriffabschnitt 4 gedreht werden.

Hierfür verrastet in der Verriegelungsposition ein distaler Zapfenabschnitt 83 des Verriegelungsschiebers 82, wie in Fig. 9 gezeigt, mit einem in der Indikatorhülse 80 aufgenommenen Rastring 84. Der Rastring 84 weist eine Mehrzahl an über den Umfang verteilten Ausnehmungen 111 auf, in welche der Zapfenabschnitt 83 in der Verriegelungsposition vorragt, um so das Bedienelement 12 in Umfangsrichtung relativ zu dem Handgriffabschnitt 4 festzulegen bzw. festzuhalten. Die Ausnehmungen des Rastrings 84 entsprechen dabei vorzugsweise den auf der Außenumfangsfläche der Indikatorhülse 80 angebrachten Indikatoren 78. Somit kann der Verwender einfach ein Abwinkeln des Werkzeugs 8 bei einem definierten Winkel einstellen und das abgewinkelte Werkzeug 8 bei diesem Winkel fixieren.

Um das Bedienelement 12 für eine Verstellung des Winkels oder zum Lösen der Kupplung relativ zu dem Handgriffabschnitt 4 drehen zu können, muss der Verriegelungsschieber 82 folglich aus der Verriegelungsposition in die Entriegelungsposition verlagert werden, d.h. in Richtung proximal.

Bei dem Motorhandstück 2 gemäß der ersten Ausführungsform ist der Verriegelungsschieber 82 in Axialrichtung über ein Federelement 86 gegen das Bedienelement 12 vorgespannt. Das Federelement 86 ist dabei so zwischen dem Verriegelungsschieber 82 und einer Anschlagsfläche des Bedienelements 12 angeordnet, dass es den Verriegelungsschieber 82 in die Verriegelungsposition, d.h. in Richtung distal, drückt. Das Federelement 86 übt folglich eine automatische Rückstellkraft auf den Verriegelungsschieber 82 aus, um diesen in der Verriegelungsposition zu halten.

Alternativ ist es selbstverständlich auch denkbar, dass das Motorhandstück 2 kein Federelement 86 aufweist. Bei einem solchen Motorhandstück gemäß einer Modifikation der ersten Ausführungsform mit manueller Rückstellung muss der Verwender den Verriegelungsschieber 82 zur Entriegelung in Richtung proximal ziehen und zur Verriegelung aktiv in Richtung distal drücken.

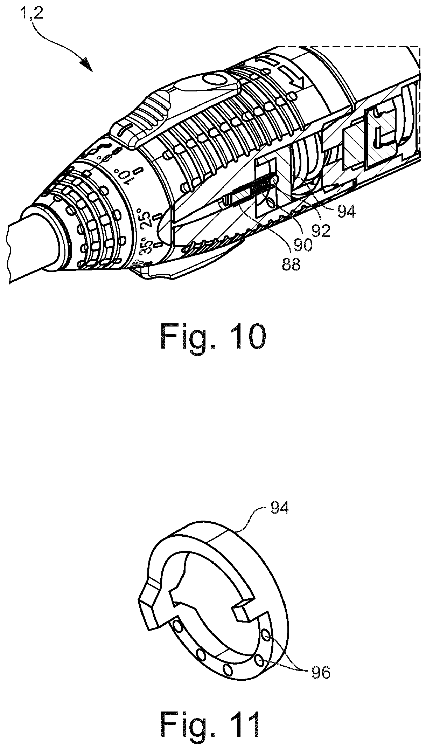

Wie in Fign. 10 und 11 gezeigt, ist bei dem Motorhandstück 2 gemäß der Modifikation der ersten Ausführungsform ein zusätzliches Kugeldruckelement 88 vorgesehen. Dieses weist ein Federelement 90 auf, welches eine Kugel 92 in Richtung proximal gegen eine fest in dem Handgriffabschnitt 4 aufgenommene Abgleitkulisse (Kalottenrastring) 94 (siehe Fig. 11) drückt. Auf der Abgleitkulisse 94 sind kalottenförmige Rastausnehmungen 96 ausgebildet, welche die Kugel 92 zumindest teilweise aufnehmen können.

Die Rastausnehmungen 96 entsprechen, ähnlich wie die Ausnehmungen des Rastrings 84, definierten Winkeln, um welche das Werkzeug 8 relativ zu dem Schaft 10 bei einer bestimmten Verdrehung des Bedienelements 12 abgewinkelt werden kann. D.h. wenn das Bedienelement 12 um den definierten Winkel relativ zu dem Handgriffabschnitt 4 verdreht wird, dreht das Kugeldruckelement 88 mit dem Bedienelement 12 mit, bis der definierte Winkel erreicht ist, bei welchem die Kugel 92 aufgrund der Vorspannkraft des Federelements 90 in die entsprechende Rastausnehmung 96 der Abgleitkulisse 94 greift. Das zusätzliche Kugeldruckelement 88 ermöglicht folglich ein haptisches Feedback für den Verwender bei der Winkeleinstellung.

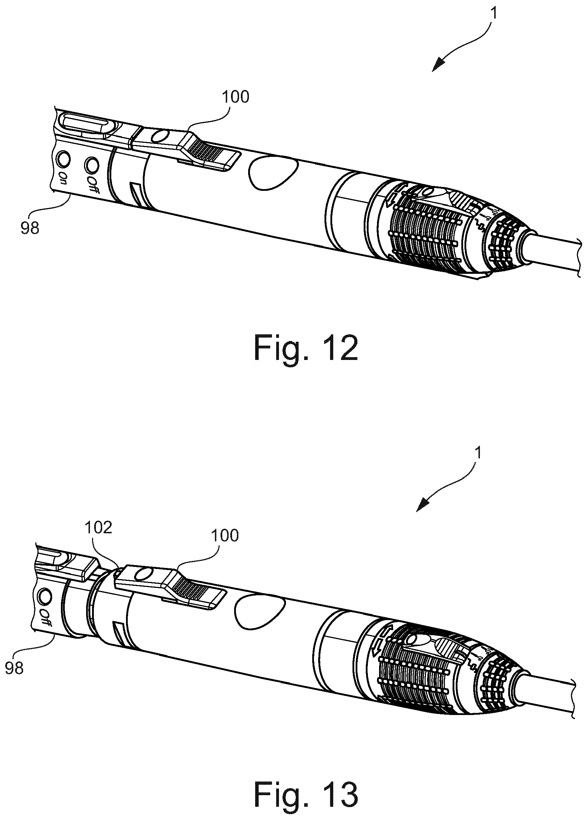

Fig. 12 zeigt das medizinische Handstück 1 gemäß der ersten Ausführungsform in einem Betriebszustand, bei welchem das Motorhandstück 2 an die Antriebseinheit 6 angeschlossen ist. Hierfür weist das Motorhandstück 2, wie vorstehend erwähnt, an seinem proximalen Endabschnitt den Anschluss auf, welcher, wie in Fig. 12 gezeigt, ein Motorkabel 98 aufnehmen kann. Das Motorhandstück 2 weist ferner einen Sperrschieber 100 auf, welcher in der OFF-Position (siehe Fig. 13) in Richtung proximal verlagert werden kann, um die Verbindung zwischen dem Schaft 10 und dem Motorhandstück 2 zu lösen.

Für die Sicherung der Entriegelungsposition des Werkzeugs 8 hat der Sperrschieber 100, wie in Fig. 13 zu erkennen, einen Sperrriegel 102. Der Sperrriegel 102 ist dabei mit dem Bedienelement 12 verbunden und wird beim Entriegeln des Werkzeugs 8 durch Drehung des Bedienelements 12 in der zweiten Drehrichtung B, d.h. beim Lösen des Wirkeingriffs zwischen der ersten Kupplungsvorrichtung 28 und der zweiten Kupplungsvorrichtung 30, in Richtung proximal ausgefahren.

Wenn das medizinische Handstück 1 in dem Betriebszustand ist, ist dieses proximale Ausfahren des Sperrriegels 102 nicht möglich, da das Motorkabel 98, insbesondere eine Nase des Motorkabels 98, ein Ausfahren sperrt. Somit kann verhindert werden, dass im Betrieb durch ein versehentliches Drehen des Bedienelements 12 in der zweiten Drehrichtung B das Werkzeug 8 ausgeworfen wird. Eine Drehung des Bedienelements 12 in der ersten Drehrichtung A bleibt jedoch weiterhin möglich, so dass das Werkzeug 8 auch im Betrieb relativ zu dem Schaft 10 abgewinkelt werden kann.

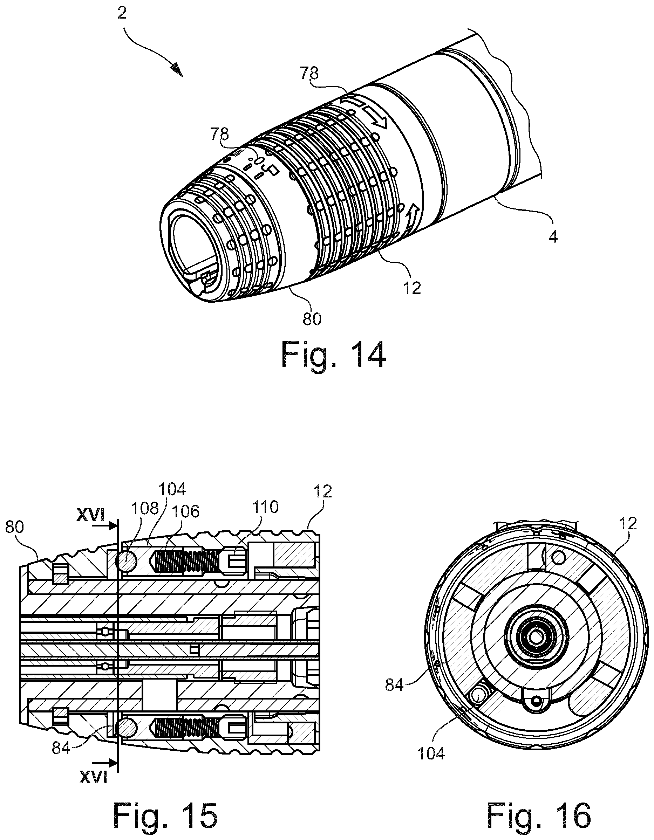

In Fig. 14 ist ein Motorhandstück 2 für ein medizinisches Handinstrument 1 gemäß einer zweiten Ausführungsform gezeigt. Dabei ist zu erkennen, dass das Motorhandstück 2 gemäß der zweiten Ausführungsform keinen Verriegelungsschieber 82 aufweist. Das Weglassen des Verriegelungsschiebers 82 ermöglicht dabei eine verbesserte Sicht auf das Operationsfeld, eine intuitivere Bedienung und einen verringerten Reinigungsaufwand.

Um dennoch, auch ohne Verriegelungsschieber 82, eine gewisse Sicherung des Bedienelements 12 gegen ungewolltes Verdrehen zu gewährleisten und eine Arretierung des Bedienelements 12 bei definierten Winkeln zu ermöglichen, sind in dem Bedienelement 12, wie in Fig. 15 gezeigt, zwei Kugeldruckelemente 104 angeordnet. Diese weisen jeweils ein Federelement 106 auf, welches eine Kugel 108 als Rastkörper gegen den Rastring (Rastblech) 84 drückt. D.h. das Federelement 106 drückt die Kugel 108 in Richtung distal gegen den Rastring 84. Dieser weist, wie in Fig. 16 zu erkennen, Ausnehmungen aus, mit welchen die Kugeln 108 verrasten.

Die Kugeldruckelemente 104 drücken, wie vorstehend erwähnt, mit ihren distalen Endabschnitten, d.h. den Kugeln 108, gegen den Rastring 84. An ihrem proximalen Endabschnitt weisen die Kugeldruckelemente 104 jeweils ein Federkrafteinstellmittel in Form einer Madenschraube 110 auf. Durch Einschrauben der Madenschraube 110 kann die Vorspannung des Federelements 106 und damit die auf den Rastring 84 wirkende Federkraft eingestellt werden. Eine höhere Federkraft erschwert ein versehentliches Verdrehen des Bedienelements 12, wohingegen eine geringere Federkraft ein leichteres Verstellen des Winkels erlaubt und damit eine Einhand-Bedienung ermöglicht.

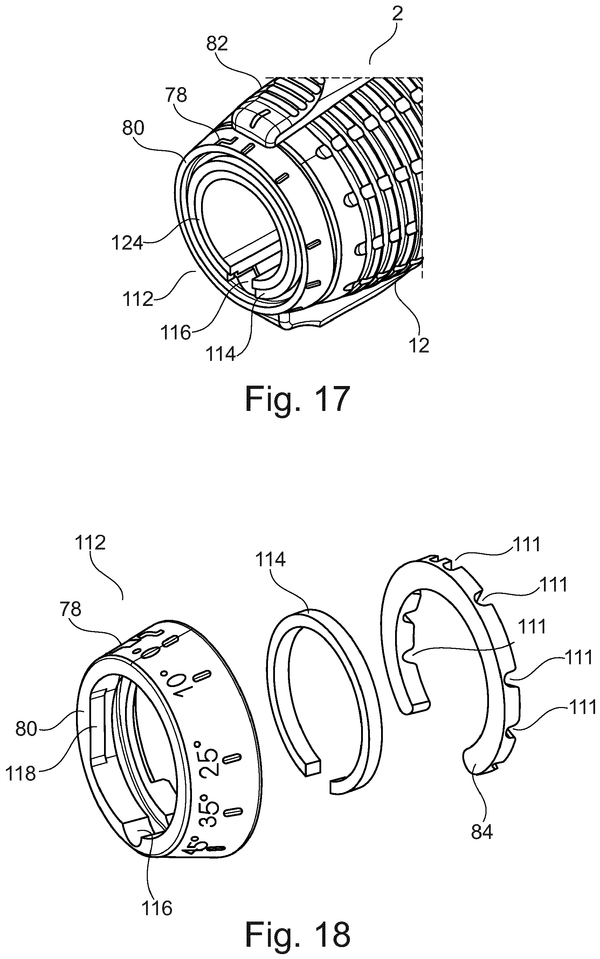

Figur 17 zeigt eine weitere Querschnittansicht des Motorhandstücks 2 des medizinischen Handinstruments 1 gemäß der ersten Ausführungsform. Die Schnittebene verläuft in der gezeigten Ansicht durch den Winkelstellring 112, senkrecht zu der Längsachse des Motorhandstücks 2. Der Winkelstellring 112 weist eine Indikatorhülse 80 auf, in deren Inneren ein Sprengring 114 angeordnet ist. Der Sprengring 114 ist dabei teilringförmig mit einem unteren Umfangsausschnitt ausgebildet und umgibt einen Träger 124 des Motorhandstücks 2. Die Indikatorhülse 80 ist konisch ausgeführt, verengt sich zum distalen Ende des Motorhandstücks 2 hin und weist auf der Mantelfläche Indikatoren auf, welche eine Winkelstellung des Bedienelements 12 angeben / anzeigen. Das Bedienelement 12 mit einem äußeren Verriegelungsschieber 82 grenzt ferner an den Winkelstellring 112 an. Außerdem ist die Einführöffnung für den Verstellstift 14 des Schafts 10 gleichzeitig als eine Demontageöffnung 116 für den Sprengring ausgebildet. Die Demontageöffnung 116 verläuft durch die Ausnehmung am (hier unteren) Rand des Sprengrings 114, durch den Träger 124 des Motorhandstücks 2.

Figur 18 zeigt eine Explosionsdarstellung des Winkelstellrings 112 des Motorhandstücks 2. Zu erkennen ist die dreiteilige Ausführung des Winkelstellrings 112, welches die äußere Indikatorhülse 80, den innenliegenden Sprengring 114 sowie den Rastring 84 aufweist. Die konische Indikatorhülse 80 weist entlang des gesamten Mantelflächenumfangs Indikatoren 78 zur Anzeige der Winkelstellung des Bedienelements 12 auf. Ferner, ist die Indikatorhülse 80 im montierten Zustand über zwei gegenüberliegende Kanten 118 gegen den Träger 124 verdrehgesichert bzw. verdrehsicherbar. Der Rastring 84 ist ähnlich zum Sprengring 114 teilringförmig ausgebildet und weist auf einer Seite eine Vielzahl an Ausnehmungen 111 auf, welche über den Umfang des Rastringes 84 verteilt angeordnet sind. Ferner, weist die Indikatorhülse 80 eine Demontageöffnung 116 für den Sprengring 114 auf, welche nutförmig durch den Innenumfang der Indikatorhülse 80, entlang der Achse des Motorhandstücks 2 verläuft.

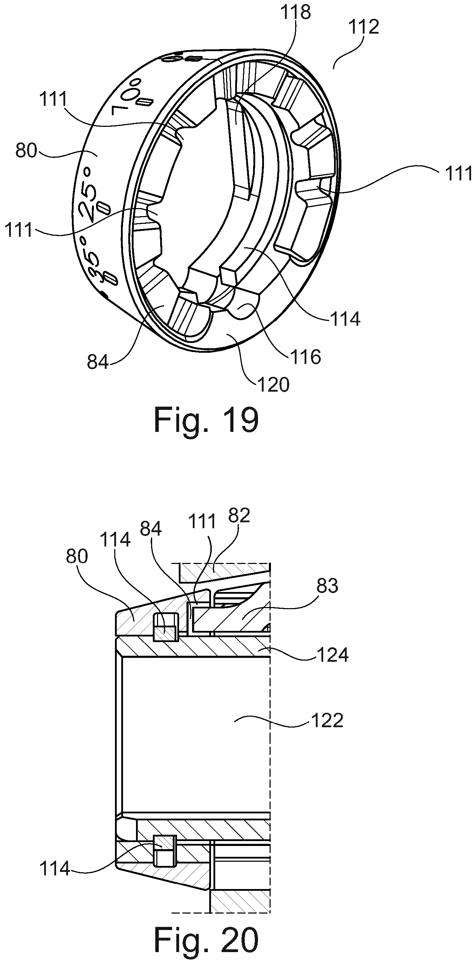

Figur 19 stellt eine isometrische Perspektivansicht auf die Rückseite 120 des Winkelstellrings 112 in einem zusammengesetzten Zustand dar. Der Sprengring 114 ist in dieser Darstellung in einer innenseitigen, umlaufenden Nut innerhalb der Indikatorhülse 80 angeordnet. Der Rastring 84 ist in eine Ausnehmung an der Rückseite der Indikatorhülse 80 eingesetzt, welche der Form des Rastringes 84 entspricht. Dabei fluchtet die Rückseite 120 der Indikatorhülse 80 mit der Rückseite des Rastringes, an welcher die Vielzahl der (Rast-)Ausnehmungen 111 angeordnet sind. Des Weiteren ist in dieser Darstellung eine Kante 118 zur Verdrehsicherung am Innenumfang der Indikatorhülse 80 sichtbar.

Figur 20 zeigt eine Teillängsschnittansicht des Motorhandstücks 2 mit dem montierten Winkelstellring 112, gemäß der ersten Ausführungsform. Die Schnittebene dieser Teilansicht verläuft entlang des Trägers 124, welcher ein Durchgangsloch 122 aufweist. Der Winkelstellring 112 ist über den innenliegenden Sprengring 114 innerhalb einer Umlaufnut am Träger befestigt. Den Sprengring 114 umgreifend ist die konische Indikatorhülse 80 angeordnet, welche außerdem den Rastring 84 an dessen Rückseite aufweist. Ein Zapfenabschnitt 83 des Bedienelements 12 ist dabei verrastend in einer der Ausnehmungen 111 des Rastringes aufgenommen. Ferner, ist ein Teilabschnitt des außenliegenden Verriegelungsschiebers 82 abgebildet.

### Bezugszeichenliste

- 1: Handinstrument
- 2: Motorhandstück
- 4: Handgriffabschnitt
- 6: Antriebseinheit
- 8: Werkzeug
- 10: Schaft
- 12: Bedienelement
- 14: Verstellstift
- 16: Axialnut
- 18: Drehübertragungshülse
- 20: proximaler Schaftabschnitt
- 21: Kante
- 22: distaler Schaftabschnitt
- 24, 26: angestellte Stirnseite
- 28: erste Kupplungsvorrichtung
- 30: zweite Kupplungsvorrichtung
- 32: Werkzeugkopf / Effektor
- 34: Werkzeugschaft
- 36: Wälzlagereinheit
- 38: Antriebswelle
- 40: Wälzlager
- 42: Lagergehäuse
- 44: Axialsicherungsnut
- 46: Verriegelungskugel
- 48: Raststift
- 50: Außenrohr
- 52: Hohlrad
- 54: Innenverzahnung
- 56: Ritzel
- 58: Außenverzahnung
- 60: Sicherungsbuchse
- 62: Hohlwelle
- 64: Aufnahmebohrung
- 66: Wälzlager
- 68: Verstellbuchse
- 70: Silikonschlauch
- 72: Raststift-Aufnahmevertiefung
- 74: Vorspannelement
- 76: Rastvorsprung
- 78: Indikator
- 80: Indikatorhülse / Markierungsring
- 82: Verriegelungsschieber
- 83: Zapfenabschnitt
- 84: Rastring / Rastblech
- 86: Federelement
- 88: Kugeldruckelement
- 90: Federelement
- 92: Kugel
- 94: Abgleitkulisse (Kalottenrastring)
- 96: Rastausnehmung
- 98: Motorkabel
- 100: Sperrschieber
- 102: Sperrriegel
- 104: Kugeldruckelement
- 106: Federelement
- 108: Kugel
- 110: Madenschraube
- 111: Ausnehmung
- 112: Winkelstellring
- 114: Sprengring
- 116: Demontageöffnung
- 118: Kante
- 120: Rückseite des Winkelstellrings
- 122: Durchgangsloch
- 124: Träger

## Patentansprüche

1. Medizinisches Motorhandstück (2) zum Antreiben eines distalen Endeffektors (8, 22), mit
einem Handgriffabschnitt (4),
einem vorzugsweise hülsenförmigen Bedienelement (12), das um eine Handgrifflängsachse drehbar an einem distalen Endabschnitt des Handgriffabschnitts (4) gehalten ist und mit dem Endeffektor (8, 22) derart koppelbar oder gekoppelt ist, um eine Drehbewegung des Bedienelements (12) in eine Bewegung des Endeffektors (8, 22) zu transformieren oder eine Funktion am Endeffektor (8, 22) zu bewirken,
**gekennzeichnet durch** einen Winkelstellring (112), welcher an einem distalen Ende des medizinischen Motorhandstücks (2) drehfest angeordnet ist und welcher derart ausgebildet und vorgesehen ist, Winkelstellungen des Bedienelements (12) verrastend festzulegen.

2. Medizinisches Motorhandstück (2) nach Anspruch 1, **dadurch gekennzeichnet, dass** der Handgriffabschnitt (4) koaxial an eine Antriebseinheit (6) ankoppelbar ist.

3. Medizinisches Motorhandstück (2) nach Anspruch 1 oder 2, **dadurch gekennzeichnet, dass** die Antriebseinheit im Motorhandstück (2) aufgenommen ist.

4. Medizinisches Motorhandstück (2) nach einem der Ansprüche 1 bis 3, **dadurch gekennzeichnet, dass** eine Mantelfläche des Winkelstellrings (112) als eine Indikatorhülse (80) mit Winkelmarkierungen ausgebildet ist.

5. Medizinisches Motorhandstück (2) nach einem der Ansprüche 1 bis 4, **dadurch gekennzeichnet, dass** der Winkelstellring (112) einen Rastring (84) aufweist, welcher in die Indikatorhülse (80) einlegbar ist und eine Vielzahl an Ausnehmungen (111) aufweist, die dafür vorgesehen sind, komplementäre Elemente des Bedienelements (12) in den jeweiligen Winkelstellungen verrastend aufzunehmen.

6. Medizinisches Motorhandstück (2) nach Anspruch 5, **dadurch gekennzeichnet, dass** der Rastring (84) ein Übermaß bezogen auf dessen Durchmesser aufweist, sodass der Rastring (84) beim Einlegen in die Indikatorhülse (80) selbsttätig in der Indikatorhülse (80) fixierbar oder fixiert ist.

7. Medizinisches Motorhandstück (2) nach einem der Ansprüche 5 oder 6, **dadurch gekennzeichnet, dass** der Rastring (84) eine konische Mantelfläche aufweist.

8. Medizinisches Motorhandstück (2) nach einem der Ansprüche 5 bis 7, **dadurch gekennzeichnet, dass** die Fläche der Indikatorhülse (80), an welche die Mantelfläche des eingelegten Rastringes (84) angrenzt, konisch ausgebildet ist.

9. Medizinisches Motorhandstück (2) nach einem der vorhergehenden Ansprüche 1 bis 8, **dadurch gekennzeichnet, dass** der Winkelstellring (112) einen innenliegenden, von außen nicht sichtbaren Sprengring (114) aufweist, mithilfe dessen der Winkelstellring (112) an einem Träger (124) des Motorhandstücks (2) axialgesichert befestigbar oder befestigt ist.

10. Medizinisches Motorhandstück (2) nach einem der vorhergehenden Ansprüche 1 bis 9, **dadurch gekennzeichnet, dass** der Rastring (84) aus einem gehärteten Stahl gefertigt ist.

11. Medizinisches Motorhandstück (2) nach einem der Ansprüche 4 bis 10, **dadurch gekennzeichnet, dass** die Mantelfläche der Indikatorhülse (80) konisch ausgebildet ist und sich im Durchmesser zum distalen Ende des Motorhandstücks (2) hin verengt.

12. Medizinisches Handinstrument (1) mit einem medizinischen Motorhandstück (2) nach einem der Ansprüche 1 bis 11, einem Werkzeugschaft (10) und einem Endeffektor (32).
